# EUROPEAN PATENT APPLICATION

(11) **EP 2 127 670 A1**
(43) Date of publication of application: **02.12.2009**
(21) Application number: 08720265.1
(22) Date of filing: 26.02.2008
(51) Int. Cl.: A61K 39/00, A61K 9/127, A61K 47/42, A61P 37/04

(54) **LIPOSOME FOR INDUCTION OF CELL-MEDIATED IMMUNITY**

(30) Priority: 28.02.2007 JP 2007085627
(71) Applicant: National University Corporation Hokkaido University, Sapporo-shi Hokkaido 060-0808 (JP)
(72) Inventor: HARASHIMA, Hideyoshi, Kita 12-jyo Nishi 6-chome, Kita-ku, Sapporo-shi, Hokkaido 060-0812 (JP); KOGURE, Kentaro, Kita 12-jyo Nishi 6-chome, Kita-ku, Sapporo-shi, Hokkaido 060-0812 (JP); NAKAMURA, Takashi, Kita 10-jyo Nishi 8-chome, Kita-ku, Sapporo-shi, Hokkaido 060-0810 (JP); MORIGUCHI, Rumiko, Kita 10-jyo Nishi 8-chome, Kita-ku, Sapporo-shi, Hokkaido 060-0810 (JP); ONO, Kohei, Kita 10-jyo Nishi 8-chome, Kita-ku, Sapporo-shi, Hokkaido 060-0810 (JP); ATTHACHAI, Homhuan, Kita 12-jyo Nishi 6-chome, Kita-ku, Sapporo-shi, Hokkaido 060-0812 (JP)
(74) Representative: Prüfer & Partner GbR European Patent Attorneys
(86) International application number: PCT/JP2008/000351
(87) International publication number: WO 2008/105174

(57) **Abstract**

Disclosed is a liposome which includes an antigenic substance therein and has, on its surface, a peptide containing several contiguous arginine residues. The liposome enables an antigen-presenting cell to achieve the selective antigen presentation through an MHC Class-I molecule, and can induce a cell-mediated immunity specifically. The liposome can also induce the antigen presentation by a matured dendritic cell.

## Description

### TECHNICAL FIELD

The present invention relates to a liposome capable of inducing cell-mediated immunity, and the liposome can be used as a vaccine against infectious diseases or cancer.

### BACKGROUND ART

In immunotherapy for virus and cancer, it is essential to induce cell-mediated immunity to encourage the immune system in individual patients to recognize and eliminate a virus-infected cell or a cancer cell as a foreign substance. In the cell-mediated immunity induced, the virus-infected cell or the cancer cell is specifically recognized and selectively killed by an antigen-specific cytotoxic T lymphocyte (CTL). In fact, CTL specifically recognizes and attacks the virus-infected cell and the cancer cell via a T-cell receptor.

A T-cell receptor of CTL is activated not by directly recognizing a specific antigen expressed on a surface of a virus-infected cell or a cancer cell, but by an MHC Class-I molecule/antigen-specific oligopeptide complex presented by an antigen-presenting cell such as a macrophage or a dendritic cell. Specifically, a specific antigen transfected by an antigen-presenting cell is decomposed by a protease complex (proteasome) in a cell to be converted into an oligopeptide. Another oligopeptide, consisting of approximately 9 to 10 amino-acid residues in the above oligopeptide, is delivered from a cytoplasm to a vesicle membrane by a transporter in antigen processing (TAP) found in a vesicle membrane, and then an antigen-specific oligopeptide, having a high affinity to an MHC Class-I molecule, combines with an MHC Class-I molecule to be presented on a surface of the antigen-presenting cell as an MHC Class-I molecule/antigen-specific oligopeptide.

Consequently, induction of cell-mediated immunity of a patient, aimed at treating and preventing cancer, viral diseases or autoimmune diseases, requires immunization of a living organism by a cancer cell expressing a specific antigen or a virus-infected cell in itself. It is also important to activate an antigen-specific CTL by expressing an MHC Class-I molecule/antigen-specific oligopeptide complex presented via the above pathway in the antigen-presenting cell after administering a specific antigen or an oligopeptide derived therefrom. However, after the specific antigen is transfected into the antigen-presenting cell, it is presented on a surface of the antigen-presenting cell as an MHC Class-II molecule/antigen-specific oligopeptide complex to normally induce humoral immune response.

Thus, in order to present an MHC Class-I molecule/antigen-specific oligopeptide on a surface of the antigen-presenting cell, a method for treating an antigen-presenting cell with an adjuvant such as extracts of a microbial cell body or mannan-coated liposome (Patent Document 1) is normally employed to induce antigen cross-presentation. Another method for inducing antigen presentation is to induce cell-mediated immunity by specifying an antigen-specific oligopeptide in an *in vitro* experiment and directly administering the oligopeptide to a patient.

However, this method involves a problem of toxicity in an adjuvant like extracts of a microbial cell body and another problem with conversion of antigen presentation by an MHC Class-II molecule to that by an MHC Class-I molecule. Specifically, this antigen presentation conversion problem is attributed to cross-presentation method for changing presentation of an MHC Class-II molecule/antigen-specific oligopeptide complex to that of an MHC Class-I molecule/antigen-specific oligopeptide in a forced manner by treating an antigen-presenting cell with an adjuvant. In fact, even if an antigen is delivered to an antigen-presenting cell, using a conventional liposome such as pH-sensitive liposome or membrane fusogenic liposome, it is difficult to selectively induce only antigen presentation through an MHC Class-I molecule, because antigen presentation is induced through an MHC Class-II molecule.

Conversely, administration of an antigen-specific oligopeptide can cause antigen-specific immune suppression or difficult acquisition of sufficient and sustainable cell-mediated immune response (Non-Patent Document 1).

Despite its significantly higher antigen presentation ability than an immature antigen-presenting cell (cell surface presenting efficiency and period of presentation in fragmented antigen), an antigen-presenting cell matured by various types of stimuli blocks mechanism for transfecting an antigenic substance intracellularly. Unfortunately, technical reports on the introduction of an antigen into a matured antigen-presenting cell, which is expected to induce cell-mediated immunity more efficiently and sustainably, have not been released.

The inventors developed a liposome having a peptide containing several contiguous arginine residues on a surface thereof (Patent Document 2) as opposed to cell-mediated immunity induction in which DNA etc. is delivered to a cell nucleus. Patent Document 2 describes nothing on an ability to induce cell-mediated immunity of the liposome.
[Non-Patent Document 1] Rosenberg et al, Nat. Med., Vol. 4, 321-327, 1998
Patent Document 1: WO92/4887
Patent Document 2: WO2005/032593

### DISCLOSURE OF THE INVENTION

### PROBLEMS TO BE SOLVED BY THE INVENTION

It is, therefore, one object of the present invention to provide a new type of pharmaceutical which is capable of selectively inducing cell-mediated immunity, without requiring an adjuvant such as extracts of a microbial cell body.

### MEANS FOR SOLVING THE PROBLEM

The inventors unexpectedly found that an MHC Class-I molecule specific antigen presentation is encouraged on an antigen-presenting cell to selectively induce cell-mediated immunity by administering an antigenic substance encapsulated with a liposome having a peptide containing several contiguous arginine residues on a surface thereof to complete each of the following inventions.

(1) A liposome having a peptide on a surface thereof, comprising several contiguous arginine residues having an encapsulated antigenic substance.

(2) The liposome according to item (1), wherein said peptide comprises 4 to 20 contiguous arginine residues.

(3) The liposome according to item (1) or (2), wherein said peptide consists of arginine residues.

(4) The liposome according to any one of items (1) to (3), wherein said peptide is modified with a hydrophobic group or a hydrophobic compound, said hydrophobic group or said hydrophobic compound is inserted by a lipid bilayer and said peptide is exposed from said lipid bilayer.

(5) The liposome according to item (4), wherein said hydrophobic group is a stearyl group.

(6) A cell-mediated immunity-inducing agent with the liposome according to any one of items (1) to (5) as an active ingredient.

(7) The cell-mediated immunity-inducing agent according to item (6), wherein an antigenic substance is a tumor antigen.

(8) The cell-mediated immunity-inducing agent according to item (7), wherein malignant neoplasm is treated.

(9) A vaccine for treating malignant neoplasm with the liposome according to any one of items (1) to (5) having an encapsulated tumor antigen as an active ingredient.

### BRIEF DESCRIPTION OF THE DRAWINGS

The above and other objects in this invention will be seen by reference to the description taken in connection with the drawings, in which:
Figure 1 is graphs indicative of induction of selective antigen presentation through an MHC Class-I molecule by a liposome in this invention;
Figure 2 is a graph indicative of transfection ability and induction of antigen presentation through an MHC Class-I molecule of a liposome in this invention in comparison with octalysine (K8) liposome;
Figure 3a is micrographs indicative of inhibition of transfection of a liposome in this invention by amiloride as a macropinocytosis inhibitor (No inhibitor on the left and inhibitor used on the right);
Figure 3b is micrographs indicative of inhibition of transfection of K8 liposome by amiloride as a macropinocytosis inhibitor (No inhibitor on the left and inhibitor used on the right);
Figure 4 is micrographs indicative of a substance encapsulated in a liposome in this invention released in a cytoplasm (octaarginine peptide (R8) liposome on the left and
K8 liposome on the right);
Figure 5 is a graph indicative of transfection of a liposome in this invention in a matured dendritic cell (DC);
Figure 6 is a graph indicative of induction of antigen presentation even on a matured DC by a liposome in this invention through an MHC Class-I molecule;
Figure 7 is a graph indicative of rise in cytotoxic activity in mice to which a liposome in this invention was administered; and
Figure 8 is a graph indicative of inhibition of proliferation of a transplanted tumor cell in mice to which a liposome in this invention having an encapsulated tumor antigen (■ denotes liposome non-administered control, ○ R8 liposome-administered group in which a tumor antigen is not encapsulated and • R8 liposome-administered group in which a tumor antigen is encapsulated).

### BEST MODE FOR CARRYING OUT THE INVENTION

A liposome in this invention is a liposome having a peptide on a surface thereof, including several contiguous arginine residues having an encapsulated antigenic substance (hereinafter referred to as polyarginine peptide).

A liposome having a polyarginine peptide in this invention on a surface thereof is a liposome described in said Patent Document 2. The number of lipid bilayers is not particularly limited if the liposome is a closed vesicle having a lipid bilayer film structure, and the liposome be a unilamellar liposome such as multilamellar vesicle (MLV), small unilamellar vesicle (SUV), large unilamellar vesicle (LUV) or giant unilamellar vesicle (GUV). Also, the liposome in this invention is not particularly limited in size, but preferably 50 to 800nm in diameter and more preferably 80 to 150nm in diameter.

In a liposome in this invention, the type of a lipid constituting a lipid bilayer is not particularly limited, but specifically one or more of phosphatidyl choline (e.g. dioleoyl-phosphatidyl-choline, dilauroylphosphatidyl-choline, dimyristoylphosphatidyl-choline, dipalmitoylphosphatidyl-choline and distearoylphosphatidyl-choline), phosphatidylglycerol (e.g. dioleoylphosphatidylglycerol, dilauroylphosphatidylglycerol, dimyristoylphosphatidylglycerol, dipalmitoylphosphatidylglycerol, distearoylphosphatidylglycerol), phosphatidyl-ethanolamine (e.g. dioleoylphosphatidylethanolamine, dilauroylphosphatidyl-ethanolamine, dimyristoylphosphatidyl-ethanolamine, dipalmitoylphosphatidyl-ethanolamine, distearoylphosphatidyl-ethanolamine), phospholipids such as phosphatidylserine, phosphatidylinositol, phosphatidic acid and cardiolipin or hydrogenated products thereof, glycolipids such as sphingomyelin and ganglioside. A phospholipids may be any one of egg yolk, soybean, or other naturally occurring lipids derived from animals or plants (e.g. egg yolk lecithin and soybean lecithin), synthetic lipid or semisynthetic lipid.

A lipid bilayer can contain one or more of animal-derived sterols such as cholesterol, cholesterol succinic acid, lanosterol, dihydrolanosterol, desmosterol and dihydrocholesterol; plant-derived sterols (phytosterol) such as stigmasterol, sitosterol, campesterol and brassicasterol; microorganism-derived sterols such as zymosterol and ergosterol; sugars such as glycerol and sucrose; glycerine fatty acid esters such as triolein and trioctanoin, so as to be physically and chemically stable and set fluidity of a membrane. A lipid bilayer content is not particularly limited, but preferably 5 to 40% to total lipids constituting the lipid bilayer (molar ratio), and more preferably 10 to 30% (molar ratio).

A lipid bilayer can contain antioxidant substances such as tocopherol, propyl gallate, ascorbyl palmitate and butylated hydroxytoluene, charged matter that provides a positive charge such as stearylamine and oleylamine, charged matter that provides a negative charge such as dicetyl phosphate, membrane proteins such as membrane extrinsic protein and membrane intrinsic protein, the content can be adjusted accordingly.

A liposome in this invention exposes a polyarginine peptide on a surface thereof. Here, "expose" means that a peptide or a peptide portion is not embedded by the lipid bilayer. Even in cases where a polyarginine peptide is not modified with a hydrophobic group or a hydrophobic compound, the polyarginine peptide is preferably exposed from an outer surface of the lipid bilayer. Also, in a multilamellar liposome, a polyarginine peptide is preferably exposed from an outer surface of each lipid membrane. However, each case doesn't rule out that a polyarginine peptide can be exposed from an inner side of a lipid membrane as well.

The number of contiguous arginine residues comprised in a polyarginine peptide is not particularly limited if it is 2 or more, but normally 4 to 20, preferably 6 to 12 and more preferably 7 to 10. The number of amino acid residues constituting the polyarginine peptide is not particularly limited, but normally 4 to 35, preferably 6 to 30 and more preferably 8 to 23. The polyarginine peptide may include any amino acid sequence added to C-terminus and/or N-terminus of several contiguous arginine residues, but preferably include only arginine residues. The most preferable polyarginine peptide is octaarginine peptide composed of only 8 arginine residues (R8).

An amino acid sequence to be added to C-terminus or N-terminus of several contiguous arginine residues is preferably a rigid amino acid sequence (e.g. polyproline). Polyproline is a linear and relatively rigid amino acid sequence, as opposed to soft and amorphous polyethylene glycol (PEG).

A ratio of a polyarginine peptide volume found on a surface of a liposome in this invention to total lipids constituting a lipid bilayer is normally 0.1 to 30% (molar ratio), preferably 1 to 25% (molar ratio) and more preferably 2 to 20% (molar ratio). If a ratio in this invention is under 2% (molar ratio), preferably under 1.5% (molar ratio) and more preferably under 1% (molar ratio), a liposome in this invention can be transferred to a cell mainly through endocytosis. A ratio of the lower limit of a polyarginine peptide volume to total lipids constituting a lipid bilayer is normally 0.1 % (molar ratio), preferably 0.5% (molar ratio) and more preferably 0.7% (molar ratio). If a ratio of a polyarginine peptide volume on a surface of a liposome in this invention to total lipids constituting a lipid bilayer is 2% or more (molar ratio), preferably 3% or more (molar ratio) and more preferably 4% or more (molar ratio), a liposome in this invention can be transferred to a cell mainly through macropinocytosis. A ratio of the upper limit of a polyarginine peptide volume to total lipids constituting a lipid bilayer is normally 30% (molar ratio), preferably 25% (molar ratio) and more preferably 20% (molar ratio).

If endocytosis mechanism determines an intracellular transitional pathway of a liposome in this invention, a lipid bilayer must include cationic lipids as a main ingredient. However, since the intracellular transitional pathway of a liposome in this invention is not actually determined only by endocytosis mechanism, the lipid bilayer doesn't have to contain cationic lipids. Consequently, a lipid bilayer of a liposome in this invention may constitute either cationic lipids or non-cationic lipids, or both. Nevertheless, cationic lipids which are prone to cytotoxicity are preferably reduced in volume contained in a lipid bilayer to remove cytotoxicity of a liposome in this invention, and a ratio of cationic lipids to total lipids constituting the lipid bilayer is preferably 0 to 40% (molar ratio) and more preferably 0 to 20% (molar ratio).

Cationic lipids are e.g., dioctadecyldimethylammonium-chloride (DODAC), N-(2,3-oleyloxy) propyl-N,N,N-trimethylammonium (DOTMA), didodecylammonium bromide (DDAB), 1,2-dioleoyloxy-3-trimethylammonio-propane, (DOTAP), 3P-N-(N',N'-dimethylaminoethane)-carbamol-cholesterol (DC-Chol), 1,2-dimyristoyloxypropyl-3-dimethylhydroxyethyl-ammonium (DMRIE) and 2,3-dioleyloxy-N-[2(sperminecarboxamido)ethyl]-N,N-dimethyl-1-propanaminum-trifl uoroacetate (DOSPA).

"Non-cationic lipid" means a neutral lipid or an anionic lipid. Neutral lipids include diacylphosphatidylcholine, diacylphosphatidyl-ethanolamine, cholesterol, ceramide, sphingomyelin, cephalin and cerebroside, and anionic lipids include cardiolipin, diacylphosphatidylserine, diacylphosphatidic acid, N-succinylphosphatidyl-ethanolamine(N-succinyl-PE), phosphatidic acid, phosphatidyl-inositol, phosphatidylglycerol, phosphatidyl-ethanediol and cholesterol succinic acid.

As a preferred embodiment in this invention, a liposome in this invention can be exemplified, in which a polyarginine peptide is modified with a hydrophobic group or a hydrophobic compound, the hydrophobic group or the hydrophobic compound is inserted into a lipid bilayer and the polyarginine peptide is exposed from a lipid bilayer. Herein, "polyarginine peptide is exposed from a lipid bilayer" means that the polyarginine peptide is exposed from either an outer surface or an inner surface of the lipid bilayer, and/or both.

A hydrophobic group or a hydrophobic compound is not particularly limited if the hydrophobic group or the hydrophobic compound can be inserted into a lipid bilayer. A hydrophobic group may be, e.g. saturated fatty acid group such as stearyl group or unsaturated fatty acid group, cholesterol group or a derivative thereof, but the saturated fatty acid group whose carbon number is 10 to 20 (e.g. palmitoyl group, oleyl group, stearyl group and arachidoyl group) is particularly preferable. The hydrophobic compound is, e.g. above-exemplified phospholipids, glycolipids or sterols, long chain aliphatic alcohols (e.g. phosphatidyl-ethanolamine and cholesterol), polyoxypropylene alkyl or glycerine fatty acid ester.

A liposome in this invention can be prepared by methods known to those of ordinary skill in the art such as hydration, ultrasonic treatment method, ethanol injection technique, ether injection technique, reverse phase evaporation method, surfactant method and freezing and thawing method. For example, in hydration, after lipids constituting a lipid bilayer and a polyarginine peptide modified with a hydrophobic group or a hydrophobic compound dissolve in an organic solvent, the organic solvent is evaporated and removed to obtain a lipid membrane. Afterward, the lipid membrane is hydrated, agitated or sonicated to produce a liposome having a polyarginine peptide on a surface thereof. Also, after lipids constituting a lipid bilayer dissolve in an organic solvent, the organic solvent is evaporated and removed to obtain a lipid membrane. Then, the lipid membrane is hydrated, agitated or sonicated to produce a liposome. Subsequently, a polyarginine peptide modified with a hydrophobic group or a hydrophobic compound is added to an external liquid of the liposome to introduce the polyarginine peptide into a surface of the liposome.

In the above method, an organic solvent can be e.g. hydrocarbons such as pentane, hexane, heptane and cyclohexane, halogenated hydrocarbons such as methylene chloride and chloroform, aromatic hydrocarbons such as benzene and toluene, lower alcohols such as methanol and ethanol, esters such as methyl acetate and ethyl acetate, and ketones such as acetone, alone or in combination with each other.

By allowing the organic solvent to pass through a filter with a predetermined pore size, a liposome having a constant particle distribution can be obtained. According to known methods, a multilamellar liposome can be converted into a single membrane liposome and a single membrane can be converted into a multilamellar liposome liposome.

An antigenic substance encapsulated in the above liposome corresponds to an antigenic substance at which cell-mediated immunity induced using a liposome in this invention is targeted. The antigenic substance is preferably an antigenic protein such as various tumor antigens, viral protein, protein derived from infectious microorganism, toxin protein and pathogenic protein.

If an antigenic substance is water-soluble, the antigenic substance can be encapsulated in an aqueous phase inside a liposome by adding the antigenic substance to an aqueous solvent used in hydration of a lipid membrane in liposome production. If an antigenic substance is lipid-soluble, the antigenic substance can be encapsulated in a lipid bilayer of a liposome by adding the antigenic substance to an organic solvent or a surfactant solution used in liposome production.

A liposome in this invention can be used e.g. as a form of dispersion. A dispersion medium can be, e.g. a buffer solution such as normal saline solution, phosphate buffer solution, citrate buffer solution or acetate buffer solution. A dispersion may be added as an additive such as sugar, polyhydric alcohol, water-soluble polymer, nonionic surfactant, antioxidant substance, pH adjusting agent or hydration accelerator.

A liposome in this invention can be used with a dispersion dried (e.g. lyophilizated, spray dried, etc.) as well. Dried liposome can be used as dispersion by adding a buffer solution, such as normal saline solution, phosphate buffer solution, citrate buffer solution or acetate buffer solution, to the dried liposome.

A liposome in this invention can be used, both *in vivo* and *in vitro.* When the liposome in this invention is used *in vivo,* the route of administration is parenteral administration such as intravenous, intraperitoneal, subcutaneous and intranasal administration. The dosage and number of administration can be adjusted according to the type and volume of an antigenic substance encapsulated in the liposome. A liposome in this invention can provide intracellular transferability in a wide range of temperature from 0 to 40°C (effectively functioning from 4 to 37°C), thereby determining temperature conditions according to purposes. If a ratio of the volume of a polyarginine peptide found on a surface of a liposome in this invention to total lipids constituting a lipid bilayer is 2% or more (molar ratio), preferably 3% or more (molar ratio) and more preferably 4% or more (molar ratio), the liposome can effectively provide intracellular transferability in a low temperature range (normally 4 to 10°C, preferably 4 to 6°C). A ratio of the upper limit of a polyarginine peptide volume to total lipids constituting a lipid bilayer is normally 30% (molar ratio), preferably 25% (molar ratio) and more preferably 20% (molar ratio).

Since a liposome in this invention, as shown in Example below, encourages an antigen-presenting cell to selectively induce only antigen presentation through an MHC Class-I molecule, the liposome can be used as an cell-mediated immunity-inducing agent. Antigen presentation through an MHC Class-I molecule induces cell-mediated immunity response achieved by the following helper T-cells, etc., and then immunologically attacks cells having an antigen presented together with the MHC Class-I molecule and foreign substances, etc. Therefore, a cell-mediated immunity-inducing agent with the liposome in this invention as an active ingredient can induce cell-mediated immunity response aimed at cells having an antigenic substance encapsulated in a liposome, such as tumor cell or virus-infected cell or an infectious microorganism, and improve an ability of immune attack against said cells of a living organism with a liposome administered.

A liposome in this invention has a property of efficiently being transfected even in a matured dendritic cell in which transfection mechanism of a foreign substance is blocked. Thus, the liposome in this invention can encourage not only an immature antigen-presenting cell but also a matured antigen-presenting cell to induce efficient and sustainable antigen presentation and cell-mediated immunity.
The present invention will be described with non-limiting Example in more detail as follows.

### EXAMPLE

### 1) Preparation of octaarginine-modified liposome having an encapsulated antigenic substance

A solution obtained by dissolving 1.116mg of DOPE, 97.34µg of EPC and 0.24mg of CHEMS (DOPE:EPC:CHEMS=7.5:1.5:1) in 0.2mL of chloroform was dispensed into glass test tubes, and nitrogen gas was blown to evaporate and dry the product to form a lipid membrane. After 1000µg of ovalbumin (OVA) as a pseudo-antigenic substance was dissolved in 10mM HEPES buffer solution, 1mg of OVA solution was added to the lipid membrane and allowed to stand at room temperature for 10 minutes to hydrate the lipid membrane. The hydrated product was sonicated (for several seconds) using an ultrasonic tank to prepare an OVA-encapsulated liposome. After adding 2mg/mL stearyl octaarginine solution to the liposome so that molar concentration to total lipids was 5mol%, the product was allowed to stand at room temperature 30 minutes to introduce octaarginine (R8) into a surface of the liposome and obtain a liposome in this invention (R8 liposome). In addition, using stearyl octalysine solution instead of stearyl octaarginine, an OVA-encapsulated liposome (K8 liposome), having an octalysine (K8) peptide on a surface of the liposome and a lipid composition (DOPE/CHEMS/EPC) identical to R8 liposome, was prepared. Also, a control liposome, having neither R8 nor K8, was prepared, using DOTAP of the same quantity as DOPE.

### 2) Confirmation of transfection of liposome to dendritic cell and antigen presentation ability

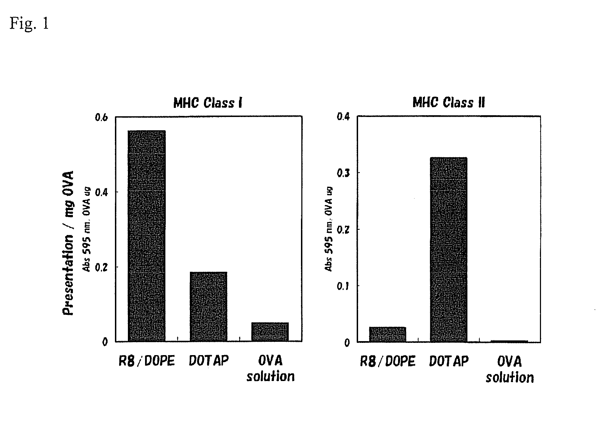

R8 liposome prepared in 1), a control liposome and an OVA which is not encapsulated in a liposome (each volume equivalent to 1.2µg of OVA) were each added to a dendritic cell (DC) (1×10⁶ cells) isolated from bone marrow of C57BL/6 mice. After each product was cultured on a serum-free medium containing 10ng/mL GM-CSF for 2 hours, 1.5my of serum medium containing 10ng/mL GM-CSF was added thereto and cultured for another 3 hours. Afterward, the cells were collected and washed on a medium of the same composition twice. Collected DCs (2×10⁵ cells) and T cells (B3Z, OTIIZ, 1×10⁵ cells) were mixed on a 96 well plate (medium volume: 0.2mL) to be cultured for 15 hours. Afterward, the cells were washed with PBS and 0.1mL of 4mM CPRG (NP-40) containing magnesium chloride was added thereto according to a Method by Shastri et al. (Shastri N. Proc. Natl. Acad. Sci. USA, Vol. 89, 6020-6024, 1992) to be incubated at 37°C for 4 hours. Then, an absorbance at a wavelength of 595nm was measured to evaluate an antigen presentation efficiency of an MHC Class-I molecule and an MHC Class-II molecule on a surface of a DC cell transfecting each liposome (Figure 1).

As a result, while only antigen presentation through an MHC Class-I molecule was confirmed in a liposome in this invention, antigen presentation through an MHC Class-II molecule was rarely found (expressed as left bars in each graph of Figure 1). Meanwhile, in the control liposome, weak antigen presentation through an MHC Class-I molecule and strong antigen presentation through an MHC Class-II molecule were confirmed.

### 3) Confirmation of specificity of octaarginine peptide

200µL of solution obtained by dissolving 1.116mg of DOPE, 0.38mg of EPC and 0.608µg of CHEMS (DOPE:EPC:CHEMS=75:23.75:1.25) in 0.2mL of chloroform was dispensed into glass test tubes, and nitrogen gas was blown to evaporate and dry the product to form a lipid membrane. 0.2mL of 10mM HEPES buffer solution containing 5mg/mL ovalbumin (OVA) as a pseudo-antigenic substance and 10mM sulforhodamine B was added to the lipid membrane and allowed to stand at room temperature for 10 minutes to hydrate the lipid membrane. The hydrated product was sonicated (for several seconds) using an ultrasonic tank to prepare an OVA-encapsulated liposome. After 2mg/mL stearyl octaarginine solution or stearyl octalysine was added to the liposome so that molar concentration to total lipids was 5mol%, the product was allowed to stand at room temperature 30 minutes to introduce octaarginine (R8) or octalysine (K8) into a surface of the liposome and prepare R8 liposome containing OVA and Sulforhodamine B and K8 liposome.

DC isolated from bone marrow of C57BL/6 mice was suspended in a serum-free medium containing 10ng/mL GM-CSF, and each of the above liposomes was added to DC of 1.5×10⁶ cells/well so that the lipid concentrations were 0, 40, 60, 80 and 100µM (final volume: 500µL/well) to be cultured under 5%CO₂ condition at 37°C for 2 hours. Then, 1.5mL of serum medium containing 10ng/mL GM-CSF was added thereto and cultured for another 3 hours. Afterward, the cells were collected and washed with a medium of the same composition twice to divide the cells into a sample for measuring volume in antigen presentation and a sample for measuring volume in transfection.

i) The sample for measuring volume in transfection was treated with 1.4mM cholic acid, and subjected to centrifugal separation (450×g) at 4°C for 5 minutes. Precipitates were suspended in 1mL of 0.1%w/v sodium azide/PBS and subjected to another centrifugal separation (450×g) at 4°C for 5 minutes. After 1mL of 1.4mM cholic acid/PBS1mL was added thereto, an extract was taken by pipetting and subjected to centrifugal separation (450×g) twice at 4°C for 5 minutes. Then, after the cells were washed with 1mL of PBS twice and suspended in PBS, they were passed through a nylon mesh and fed to an FACS tube for FACS measurement.

ii) The sample for antigen presentation was subjected to centrifugal separation (450×g) at 4°C for 5 minutes and suspended in a serum medium (1×10⁶ cells/mL). Afterward, DC of 2×10⁵ cells and S3Z of 1×10⁵ cells were placed on a 96 well plate and co-cultured under 5%CO₂ condition at 37°C for 15 hours. After collecting supernatants of co-cultured DC and B3Z in a microtube, washing each well with 200µL of PBS and collecting a washing liquid in a microtube, 50µL of chlorophenol red β-D-galactopyranoside (CPRG) buffer solution was added to each well. After the product was subjected to centrifugal separation (2000rpm) at 4°C for 5 minutes, the cells were resuspended with 50µL of CPRG buffer solution and incubated under the light shielding condition at 37°C for 4 hours to measure an absorbance at a wavelength of 595nm.

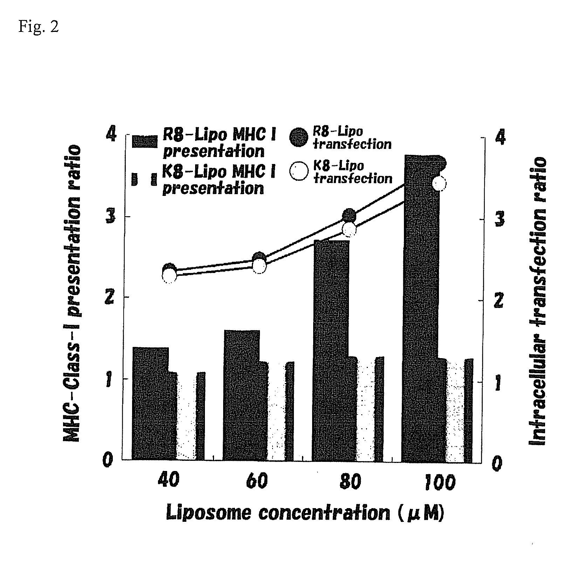

From the above results, both liposomes showed the identical volume transfected to DC, but antigen presentation through an MHC Class-I molecule was found only in R8 liposome, not in K8 liposome (Figure 2).

### 4) Confirmation of transfection pathway of liposome

200µL of solution obtained by dissolving 1.2mg of EPC, 0.022mg of cholesterol (Chol), 0.608µg of CHEMS (EPC:Chol:CHEMS=75:28.75:1.25) in 0.2mL of chloroform was dispensed into glass test tubes, and nitrogen gas was blown to evaporate and dry the product to form a lipid membrane. 0.2mL of 10mM HEPES buffer solution containing 10mM sulforhodamine B was added to the lipid membrane and allowed to stand at room temperature for 10 minutes to hydrate the lipid membrane. The hydrated product was sonicated (for several seconds) using an ultrasonic tank to prepare a sulforhodamine B-encapsulated liposome. After 2mg/mL stearyl octaarginine solution or stearyl octalysine was added to the liposome so that molar concentration to total lipids was 5mol%, the product was allowed to stand at room temperature 30 minutes to introduce octaarginine (R8) or octalysine (K8) into a surface of the liposome and prepare R8 liposome containing Sulforhodamine B and K8 liposome.

DC isolated from bone marrow of C57BL/6 mice was suspended in a serum-free medium containing 10ng/mL GM-CSF. Then, amiloride was added to DC of 1.0×10⁶ cells/well so that final concentration was 2mM and by adjusting final volume at 500µL, the product was incubated under 5%CO₂ condition at 37°C for 30 minutes. Afterward, each of the above liposomes was added so that lipid concentration was 50µM and the product was incubated under 5%CO₂ condition at 37°C for 1 hour. After 1.0mL of serum medium containing 10ng/mL GM-CSF was added thereto and an extract was taken by pipetting, the cells were subjected to centrifugal separation to be collected, and 1mL of 1.4mM cholic acid/PBS was added thereto and an extract was taken by pipetting. The product was allowed to stand at room temperature for 3 minutes and washed with PBS to be fed to an FACS tube through a nylon mesh. After FACS measurement, the cells were cultured for 3 hours. Afterward, the cells were collected and washed with a medium of the same composition twice to confirm a sample under microscopic observation using Achroplan 6.3x/0.9 W Ph3.

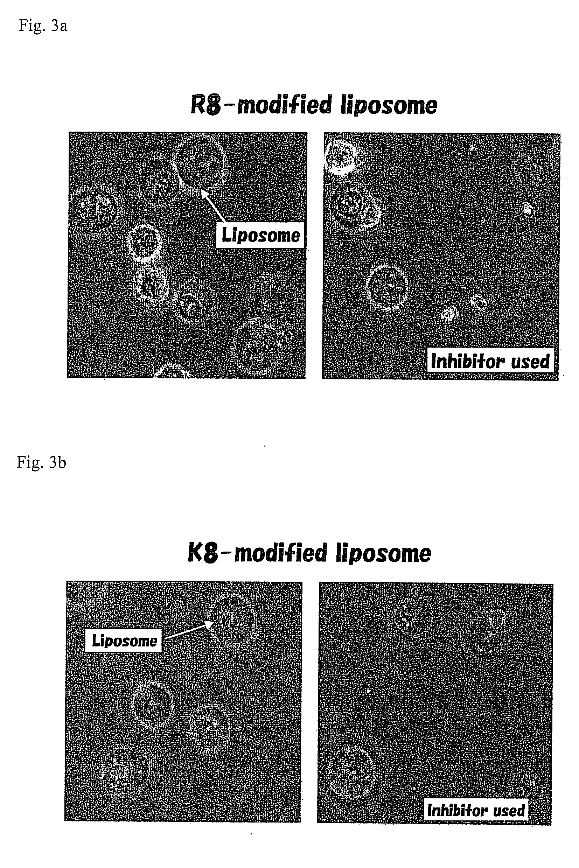

If amiloride as a macropinocytosis inhibitor coexists with both liposomes, transfection was significantly inhibited therein (expressed by right photo in Figures 3a and 3b). Therefore, it is suggested that both R8-modified liposome and K8-modified liposome can be transfected by the identical mechanism.

### 5) Confirmation of ability of liposome to release encapsulated substance

200µL of solution obtained by dissolving 1.116mg of DOPE, 0.38mg of EPC, 0.608µg of CHEMS (DOPE:EPC:CHEMS=75:23.75:1.25) in 0.2mL of chloroform was dispensed into glass test tubes, and nitrogen gas was blown to evaporate and dry the product to form a lipid membrane. 0.2mL of 10mM HEPES buffer solution containing 0.25mg/mL rhodamine-labeled dextran (70kDa) was added to the lipid membrane and allowed to stand at room temperature for 10 minutes to hydrate the lipid membrane. The hydrated product was sonicated (for several seconds) using an ultrasonic tank to prepare a rhodamine-labeled dextran-encapsulated liposome. After 2mg/mL stearyl octaarginine solution or stearyl octalysine was added to the liposome so that molar concentration to total lipids was 5mol%, the product was allowed to stand at room temperature 30 minutes to introduce octaarginine (R8) or octalysine (K8) into a surface of the liposome and prepare R8 liposome containing rhodamine-labeled dextran and K8 liposome.

After DC was collected like in 4) and the above liposome was added so that concentration was 100µM (final volume: 500µL), the product was incubated under 5%CO₂ condition at 37°C for 2 hours. A serum medium containing 10ng/mL GM-CSF was added thereto to set the total volume at 1mL. The product was incubated under 5%CO₂ condition at 37°C for 16 hours, and 100µL of cell suspension was seeded on a glass bottom dish and incubated under 5%CO₂ condition at 37°C for 20 minutes. After the cells were washed, 100µL of serum-free medium and 0.5µL of 10µM LysoTracker™ were added thereto and observed under a confocal laser scanning microscope (Lens: Plan-Apochromat 63×/1.4 Oil DIC).

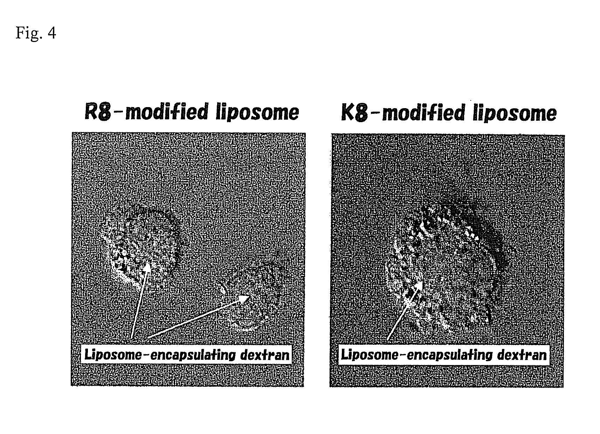

In all cells which transfected both liposomes, the cytoplasm was red-stained and dextran encapsulated in the liposome was released in the cytoplasm and diffused (Figure 4).

### 6) Transfection of liposome into matured DC

DCs (5×10⁵) differentiation-induced with 1mL of RPMI 1640 medium containing 10% fetal calf serum, PS and 10ng/mL GM-CSF for 7 days isolated from mouse bone marrow were seeded on a culture plate and 1µg/mL CpG-oligo DNA was added thereto as an adjuvant. Cells were collected in different elapsed hours (0, 2, 5, 11, 23 and 47 hours later) and washed with PBS twice. Afterward, the cells were suspended in 0.5mL of serum-free medium containing R8 liposome containing sulforhodamine B prepared in 4) and K8 liposome so that final lipid concentration was each 0.1mM and incubated at 37°C for 1 hour. After incubation was completed, the medium was removed and washed with buffer for flow cytometry (PBS containing 0.5% fetal calf serum and 0.1% NaN₃) three times. After washing, the cells were suspended in 0.5mL of buffer for flow cytometry, and passed through a nylon mesh. Afterward, a volume of fluorescence contained in a cell was measured using flow cytometer (BD Biosciences).

DC immunophenotype analysis was performed using flow cytometer for CD86 as matured marker. After DC was washed with buffer for flow cytometry twice and suspended in the same buffer, excess quantity of control monoclonal antibody (IgG1κ) was added thereto and incubated for 30 minutes to block bonding of non-specific antibody. Afterward, the cells were washed with buffer for flow cytometry twice and FITC-bonded anti-mouse CD86 antibody was added thereto and incubated in a dark place at 4°C for 30 minutes. Then, the cells were washed with buffer for flow cytometry twice and a volume of cell fluorescence was measured by flow cytometer like the above procedure and analyzed using CellQuest Software (BD Biosciences). DC maturation was evaluated based on CD86 relative expression level. As an isotype control, anti-rat CD86 (IgG2a) was employed.

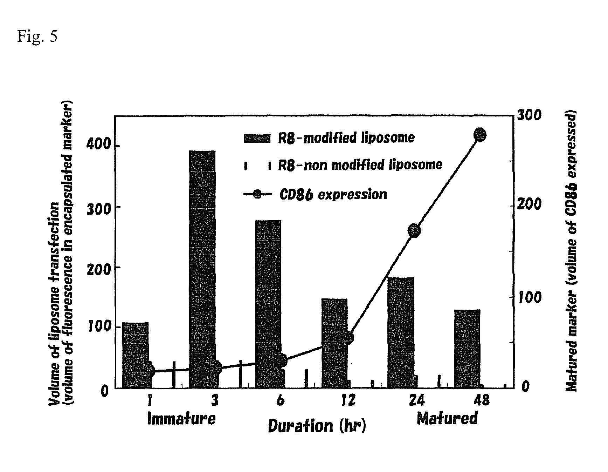

As a result, while the volume of R8 non-modified liposome transfected declines as DC becomes mature, R8 liposome already showed significantly higher transfection volume from immature state than R8 non-modified liposome, indicating a constant volume after even DC maturation (Figure 5).

### 7) Antigen presentation in matured DC

2µg/mL OVA solution and R8 liposome having an encapsulated OVA equivalent thereto in volume were each added to DC (1.2×10⁶ cells) differentiation-induced like in 6) and incubated at 37°C for 2 hours. The cells were washed with PBS twice and 2mL of RPMI 1640 medium containing 10% fetal calf serum, PS and 50µM mercaptoethanol was added thereto. To induce final maturation, 1µg/mL CpG-oligo DNA was added thereto and cultured for 22 hours.

2µg/mL OVA solution and R8 liposome encapsulated having an encapsulated OVA equivalent thereto in volume were each added to matured DC like in 6) and incubated at 37°C for 2 hours and washed with PBS twice. DC of 2×10⁵ cells/well and B3ZT cells of 1×10⁵ cells/well were co-cultured on a flat-bottom 96 well plate overnight and then the cells were washed with 0.1mL of PBS. 0.1mL of 5mM color forming lacZ substrate solution (PBS containing CPRG and 0.5% NP40) was added to a cell-dissolved solution and incubated at 37°C for 4 hours. An absorbance at a wavelength of 595nm was measured to evaluate lacZ activity.

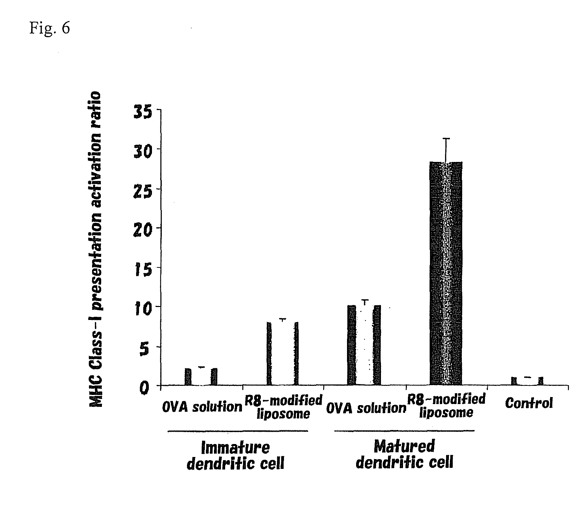

As a result, not only R8 liposome transfected in an immature DC but also matured DC showed significantly high antigen presentation. Compared to transfection of only model antigen, high antigen presentation was confirmed in transfection of R8 liposome (Figure 6).

### 8) Cell-mediated immunity induction

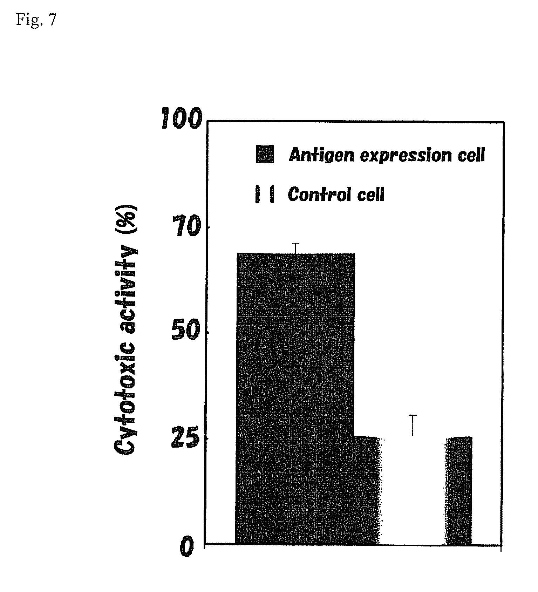

The cells obtained by adding OVA-encapsulated R8 liposome to a matured DC using an adjuvant (CpG) like in 6) and incubating for 24 hours were subcutaneously administered to C57BL/6 mice. DC stimulated with R8 liposome every one week twice was administered to the mice and one week later using OVA antigen expression cell (EG-7) and control cell (EL-4) as a target cell (E:T=200: 1), cytotoxic activity (CTL activity) was evaluated by chromium release assay. Consequently, highly specific CTL activity (approximately 40%) was found (Figure 7).

### 9) Immunity induction to virtual tumor antigen

An octaarginine liposome (R8 liposome) encapsulated with 40µg of OVA like in said 1) was prepared. 660 nmol of the liposome was subcutaneously administered to a flank of C57BL/6 mouse (female: 7 age in week) every one week twice, and one week later mouse tumor cells (8×10⁵, EG.7-OVA, ATCC) which express OVA as virtual tumor antigen were subcutaneously inoculated. Afterward, the size of tumor was continuously measured. On the other hand, 660nmol R8 liposome in which OVA is not encapsulated was prepared. EG.7-OVA (8×10⁵) was inoculated in C57BL/6 mice in which the above 660nmol R8 liposome was subcutaneously administered to a flank every one week twice and C57BL/6 mice in which nothing was administered as a control in an experiment to continuously measure the size of tumor.

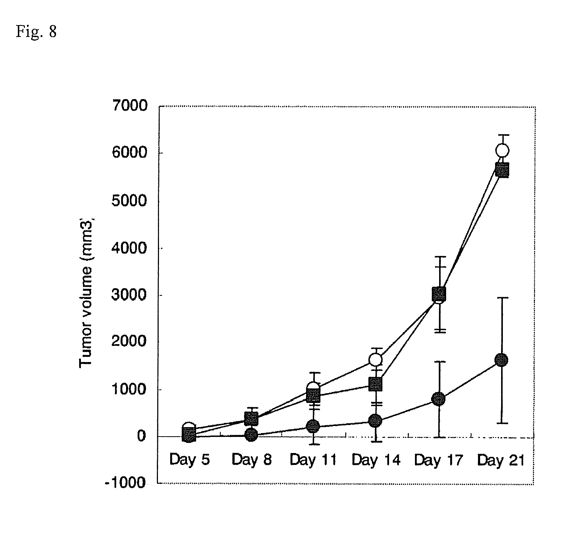

Consequently, a tumor cell significantly proliferated in nothing-encapsulated R8 liposome and nothing-administered control group, but a group in which OVA-encapsulated R8 liposome was administered showed control of tumor cell proliferation (Figure 8). From these observations, R8 liposome induces cell-mediated immunity to an encapsulated antigen, and can be used as a vaccine against cancer.

### INDUSTRIAL APPLICABILITY

A liposome in this invention enables an antigen-presenting cell to achieve the selective antigen presentation through an MHC Class-I molecule, and can induce a cell-mediated immunity specifically. Also, the liposome in this invention can encourage a matured dendritic cell to transfect an antigen.

While antigen presentation is induced through an MHC Class-II molecule only by delivering an antigen to a cytoplasm using known pH-sensitive liposome and membrane fusogenic liposome, the liposome in this invention can induce only antigen presentation through an MHC Class-I molecule specifically. Thus, the liposome in this invention can be used as a cell-mediated immunity-inducing agent. Additionally, the liposome in this invention can be efficiently transfected even in a matured dendritic cell in which transfection mechanism of an antigenic substance is blocked, enabling a matured antigen-presenting cell to induce efficient and sustainable antigen presentation.

## Claims

1. A liposome having a peptide on a surface thereof, comprising several contiguous arginine residues having an encapsulated antigenic substance.

2. The liposome as set forth in Claim 1, wherein said peptide comprises 4 to 20 contiguous arginine residues.

3. The liposome as set forth in Claim 1 or 2, wherein said peptide consists of arginine residues.

4. The liposome as set forth in any one of Claims 1 to 3, wherein said peptide is modified with a hydrophobic group or a hydrophobic compound, said hydrophobic group or said hydrophobic compound is inserted by a lipid bilayer and said peptide is exposed from said lipid bilayer.

5. The liposome as set forth in Claim 4, wherein said hydrophobic group is a stearyl group.

6. A cell-mediated immunity-inducing agent with the liposome as set forth in any one of Claims 1 to 5 as an active ingredient.

7. The cell-mediated immunity-inducing agent as set forth in Claim 6, wherein an antigenic substance is a tumor antigen.

8. The cell-mediated immunity-inducing agent as set forth in Claim 7, wherein malignant neoplasm is treated.

9. A vaccine for treating malignant neoplasm with the liposome as set forth in any one of Claims 1 to 5 having an encapsulated tumor antigen as an active ingredient.
